# EUROPEAN PATENT SPECIFICATION

(11) **EP 4 051 207 B1**
(45) Date of publication and mention of the grant of the patent: **10.07.2024**
(21) Application number: 20793701.2
(22) Date of filing: 21.10.2020
(51) Int. Cl.: A61J 1/20

(54) **SAFETY ASSEMBLY FOR THE RECONSTITUTION, THE TAKING AND THE INFUSION OF PHARMACOLOGICAL LIQUIDS**
SICHERHEITSANORDNUNG ZUR REKONSTITUTION, AUFNAHME UND INFUSION VON PHARMAKOLOGISCHEN FLÜSSIGKEITEN
ENSEMBLE DE SÉCURITÉ POUR LA RECONSTITUTION, LA PRISE ET LA PERFUSION DE LIQUIDES PHARMACOLOGIQUES

(30) Priority: 31.10.2019 IT 201900020206
(43) Date of publication of application: 07.09.2022
(73) Proprietor: Nacatur International Import Export S.r.l., 61040 Monte Porzio (PU) (IT)
(72) Inventor: NAVARRA, Stefana, 61040 Monte Porzio (IT)
(74) Representative: Baldi, Claudio
(86) International application number: PCT/EP2020/079661
(87) International publication number: WO 2021/083768

(56) References cited:
- WO-A1-2008/153460
- US-A- 4 768 568
- US-A1- 2011 015 566
- US-A1- 2015 297 839
- US-A1- 2018 200 498

## Description

The present invention relates to a safety assembly for the reconstitution, the taking and the infusion of pharmacological liquids, in particular antiblastic and/or hazardous medicines. The sector of reference is health safety, in particular the safety of health operators and patients during the preparation, the administration or the manipulation of potentially toxic pharmacological liquids.

Antiblastic and/toxic medicines are known, which are potentially hazardous for human health, for the patients and for the health operators (chemists, doctors and nurses) who manipulate said medicines on a daily basis. Health operators and patients suffer a severe risk of accidental exposure to antiblastic medicines during preparation and administration.

Generally speaking, antiblastic medicines for intravenous use need to be reconstituted, if they are lyophilized, or diluted, if they are in a concentrated solution, or taken from the bottle, if they are ready, and infused by means of couplings to access points along infusion lines.

In particular, the medicine is sucked with a syringe and is introduced in a bag of physiological saline or extemporaneous bolus in order to be administered to the patient. In general, medicines are transferred from the bottle to the infusion line by means of needle-less syringes connected with connection devices provided with first coupling means for coupling with the syringe and second coupling means for coupling with the preparation line and finally with the infusion line.

However, when using said connection devices, the health operators who are in charge of manipulation may be accidentally exposed to the chemotherapeutical medicines because of possible leaks during the uncoupling, the transfer and the coupling of the needle-less syringe/access assembly.

Such an inconvenience may involve a contamination risk for health operators and patients both during preparation and administration.

The medicines to be administered are generally prepared in a remote place relative to the location of the patient and the infusion line; therefore, during the transportation from the place of preparation to the place of infusion, a portion of the liquid/medicine may leak from the connection device, thus exposing operators or patients to a contamination risk.

US2015/297839 and US2018/200498 disclose an adapter suitable for being connected to the tip of a needle syringe and to a patient device. A first membrane is disposed in the adapter and a second membrane in the patient device. The two membranes are closed and pierced by the needle of the syringe when the adapter is coupled with the syringe and with the patient device. Obviously, such an adapter does not work with a needle-less syringe.

US4768568 discloses a protection apparatus suitable for being coupled with an opening of a vial. Said protection apparatus has two closed membranes that are pierced by a needle of a syringe.

WO2008/153560 discloses a medical device for the supply of gas provided with a closed membrane disposed above a filter. The tip of a needle pierces the membrane in order to inject the gas in the filter disposed under the membrane.

US2011/015566 discloses an adapter for a needle-less syringe. Such an adapter comprises a first membrane with a hole, a second membrane with a hole and a cannula disposed between the two membranes. The syringe has a head that compresses the first membrane, enlarging the hole of the first membrane to let the liquid pass through, and pushes the cannula that compresses the second membrane, enlarging the hole of the second hole to let the liquid pass through. It must be noted that the adapter is not provided with coupling means for coupling with a syringe and a patient device. The forward movement of the syringe head towards the adapter causes a compression of the two membranes, piercing the holes of both membranes. Therefore, said adapter is impaired by the fact that the medicine may leak during the transportation of the syringe/adapter assembly if the syringe is accidentally actuated when the adapter is not yet coupled with a patient device.

The purpose of the present invention is to overcome the drawbacks of the prior art by disclosing an assembly for the reconstitution, the taking and the infusion of pharmacological liquids that is reliable, inexpensive and extremely safe, and prevents the liquid from leaking during the coupling or the uncoupling of the safety device with a syringe disposed upstream and with a patient device disposed downstream.

These purposes are achieved according to the invention with the characteristics of the appended independent claim 1.

Advantageous embodiments appear from the dependent claims.

The safety assembly according to the invention is defined by claim 1.

For the sake of clarity, the description of the assembly according to the invention continues with reference to the appended drawings, which have a merely illustrative, not limiting value, wherein:

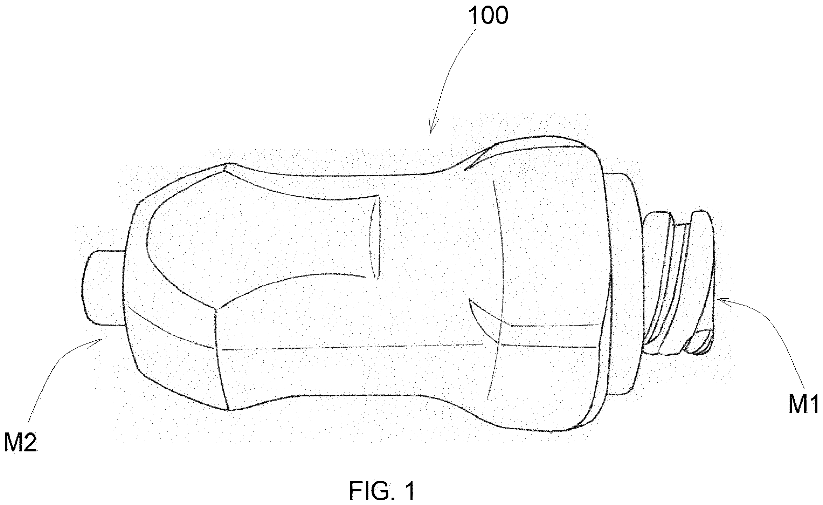

Fig. 1 is a side view of the safety device of the assembly according to the invention;
Fig. 2 is an exploded axial view of the safety device of the assembly according to the invention;
Fig. 3 is an axial view of the safety device, wherein the coupling means of the safety device are not coupled;
Fig. 4 is an axial view of the safety device, wherein the coupling means of the safety device are coupled with corresponding coupling means;
Fig. 5 is a diagrammatic side view of a needle-less syringe connected to the safety device;
Fig. 6 is a diagrammatic side view of the safety device connected to a needle-less syringe disposed upstream the safety device and to a patient device disposed downstream the safety device, wherein the patient device has a needle;
Fig. 7 is a diagrammatic side view of the safety device connected to a patient device, wherein the patient device is connected to a bottle.

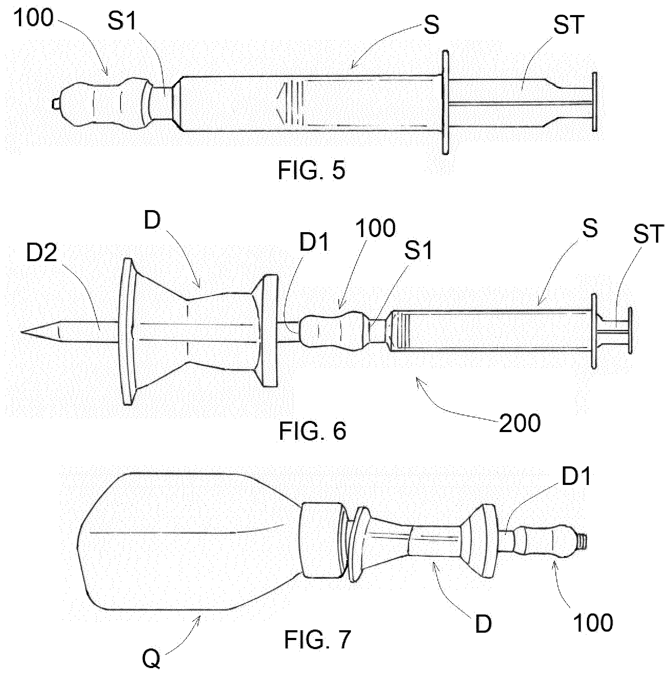

With reference to Fig. 6, a safety assembly for the reconstitution, the taking and the infusion of pharmacological liquids, which is generally indicated with reference numeral (200), is disclosed. The safety assembly is suitable for being especially used with antiblastic and/or hazardous pharmacological liquids.

The safety assembly (200) comprises: a safety device (100), a syringe (S) and a user device (D). The safety device (100) is suitable for being coupled with the syringe (S) and the user device (D).

The user device (D) may have a needle (D2) for the injection of the pharmacological fluid or the user device (D) can be connected to a receptacle (Q) (Fig. 7), such for example a vial, a bottle, or a bag, for the administration/infusion of the pharmacological fluid.

With reference to Figs. 5 and 6, the syringe (S) is a needle-less syringe. The syringe (S) has a head comprising coupling means (S1) suitable for coupling with the safety device (100).

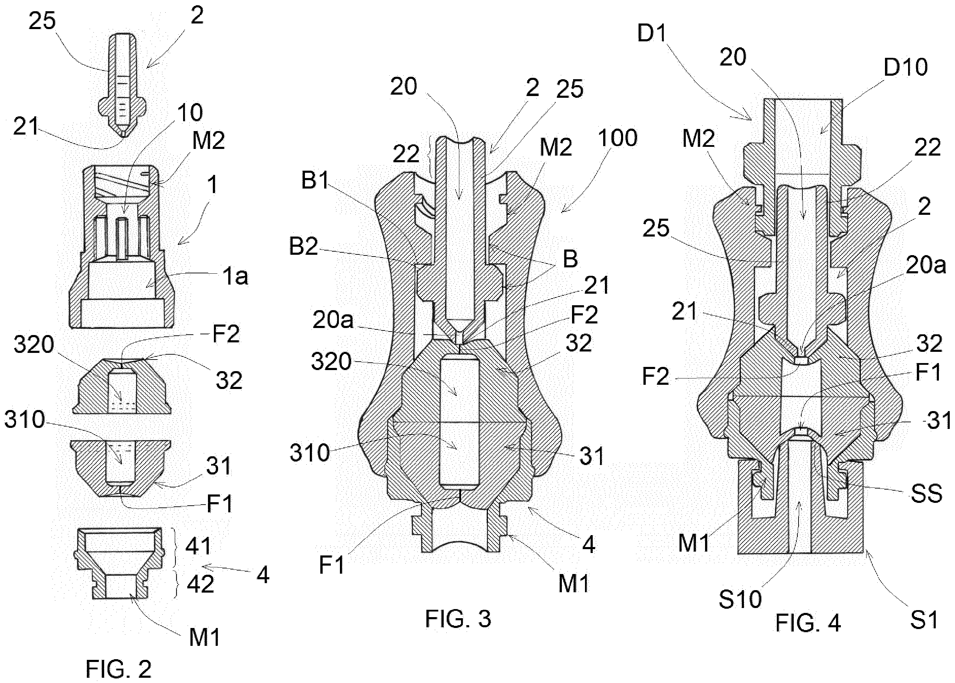

With reference to Fig. 4, the syringe (S) comprises an axial duct (S10) with an opening obtained in correspondence of said coupling means (S1).

With reference to Figs. 6 and 7, the user device (D) comprises coupling means (D1) suitable for coupling with the safety device (100).

With reference to Fig. 4, the user device (D) comprises an axial duct (D10) with an opening obtained in correspondence of said coupling means (D1).

The user device (D) is suitable for being hydraulically connected to the receptacle (Q) for taking or injecting a pharmacological liquid. The user device (D) may be a spike, a tube, an injection system or the like.

With reference to Figs. 2, 3 and 4, the safety device (100) comprises a body (1) with concave external walls to favor the grip of the safety device (100).

The safety device (100) comprises:
- first coupling means (M1) configured in such a way to be coupled with the coupling means (S1) of the syringe (S) in such a way that the syringe is fixed to the safety device; and
- second coupling means (M2) configured in such a way to be coupled with the coupling means (D1) of the user device (D), in such a way that the user device is fixed to the safety device.

The first coupling means (M1) and the second coupling means (M2) of the safety device are disposed at opposite ends.

The safety device (100) comprises an axial duct (10) obtained along the body (1) that extends from the first coupling means (M1) to the second coupling means (M2).

When the first coupling means (M1) of the safety device (100) are coupled with the coupling means (S1) of the syringe (S), the axial duct (10) of the safety device (100) and the axial duct (S10) of the syringe are coaxial.

When the second coupling means (M2) of the safety device (100) are coupled with the coupling means (D1) of the user device (D), the axial duct (10) of the safety device (100) and the axial duct (D10) of the user device (10) are coaxial.

Advantageously, the coupling means (S1) of the syringe (S) comprise a luer-lock male connector, the coupling means (D1) of the user device (D) comprise a luer-lock female connector; the first coupling means (M1) of the safety device (100) comprise a luer-lock female connector; and the second coupling means (M2) of the safety device (100) comprise a luer-lock male connector.

With reference to Figs. 2, 3 and 4, the safety device (100) also comprises a first membrane (31) and a second membrane (32) disposed inside the axial duct (10). Each membrane (31, 32) may be in a non-compressed closed position, wherein the membrane (31, 32) obstructs the axial duct (10), and in a compressed open position, wherein the membrane (31, 32) does not obstruct the axial duct (10).

The first membrane (31) is normally closed and may be compressed to an open position when the coupling means (S1) of the syringe (S) are coupled with the first coupling means (M1) of the safety device (100).

The second membrane (M2) is normally closed and may be compressed to an open position when the coupling means (D1) of the user device (D) are coupled with the second coupling means (M2) of the safety device (100).

The safety assembly (200) comprises:
- first compression means (SS) associated with the coupling means (S1) of the syringe that compress the first membrane (31) to the compressed open position, when the coupling means (S1) of the syringe are coupled with the first coupling means (M1) of the safety device (100); and
- second compression means (2) associated with said coupling means (D1) of the user device that compress the second membrane (32) to the compressed open position, when the coupling means (D1) of the user device are coupled with said second coupling means (M2) of the safety device (100).

The second compression means (2) compress the second membrane (32) in such a way to cause its deformation in the open position. The second compression means (2) comprise a cannula (25) that is mounted with possibility of axially sliding inside the axial duct (10) of the safety device (100).

The cannula (25) may slide from a non-operating position (Fig. 3), wherein the second membrane (32) is not compressed, to an operating position (Fig. 4), wherein the second membrane (32) is compressed, when said coupling means (D1) of the user device (D) are coupled with the second coupling means (M2) of the safety device (100).

The cannula (25) comprises a tip (21) that pushes the second membrane (32) to its open position, when the cannula (25) is in its operating position. The cannula (25) comprises a through axial duct (20) and a hole (20a) obtained in the tip (21).

With reference to Fig. 3, when the coupling means (D1) of the user device (D) are not yet coupled with the second coupling means (M2) of the safety device (100), the tip (21) of the cannula rests on the second membrane (32), without compression, and an ending portion (22) of the cannula externally protrudes from the axial duct (10) of the body of the safety device, in correspondence of the second coupling means (M2) of the safety device (100).

With reference to Fig. 4, when the coupling means (D1) of the user device (D) are coupled with the second coupling means (M2) of the safety device (100), the ending portion (22) of the cannula (25) is coupled by interference inside the axial duct (D10) of the user device (D). In such a way, the user device pushes the cannula (25) to its operating position towards the second membrane (32), with compression.

With reference to Fig. 4, the first compression means (SS) comprise a tip of the coupling means (S1) of the syringe (S) suitable for pushing the first membrane (31) in such a way to compress the first membrane (31) to its open position, when said coupling means (S1) of the syringe (S) are coupled with the first coupling means (M1) of the safety device (100).

With reference to Fig. 3 and 4, each membrane (31, 32) comprises a central hole (F1, F2) that, when said membrane (31, 32) is in closed position, is suitably dimensioned to prevent a passage of a liquid and therefore the axial duct (10) of the safety device is obstructed. On the contrary, when the membrane (31, 32) is disposed in said open position, said central hole (F1, F2) is enlarged, generating a through opening that permits the passage of a liquid, as shown in Fig. 4.

With reference to Fig. 2, 3 and 4, the safety device (100) is provided along said axial duct (10) with a seat (1a) suitable for receiving the two membranes (31, 32).

The two membranes (31, 32) are identical and opposite. More precisely, the two membranes (31, 32) are shaped like a semi-body disposed in mutual contact, and provided with ducts (310, 320) in coaxial position and in communication with the holes (F1, F2). The duct (310, 320) of each membrane (31, 32) ends in the duct (320, 310) of the other membrane (32, 31) on one side, and in the hole (F1, F2) of the membrane (31, 32) on the other side.

The safety device (100) also comprises a lid (4) that comprises a first portion (41) inserted in the axial duct (10) of the safety device (100), and a second portion (42) that comprises said first coupling means (M1) of the safety device (100).

The seat (1a) that houses the two membranes (31, 32) is defined by the first portion (41) of the lid (4) and by a portion of the axial duct (10) of the safety device (100).

Preferably, the second membrane (32) constantly pushes the cannula (25) to its non-operating position. More precisely, the second membrane (32) comprises elastic elements that tend to bring the second membrane (32) to its closed position, consequently pushing the cannula (25) to its non-operating position. So, the second membrane (32) acts a pushing means for the cannula (25). Alternatively, the safety device (100) may comprise pushing means to push the cannula (25) to its non-operating position. For illustrative purposes, said pushing means may consist in a helical spring disposed around the cannula (25) to push the cannula to its non-operating position.

With reference to Figs. 3 and 4, the safety device (100) comprises stop means (B) suitable for stopping said cannula (25) in its non-operating position to prevent the cannula from completely coming out from the axial duct (10) of the safety device (100). The stop means (B) comprise a stop tooth (B1), which is preferably shaped like an annular collar obtained in the longitudinal duct (10) that protrudes radially outwards, and a plurality of radial tabs (B2) obtained around said cannula that protrude radially outwards. The radial tabs (B2) are suitable for being stopped against the stop tooth (B1) when the cannula (25) is in non-operating position.

Following is a description of the use and the operation of the safety device (100).

Firstly, a needle-less syringe (S) with coupling means (S1) is provided, which is connected to the safety device (100) with the first coupling means (M1), as shown in Fig. 5. In view of the above, the first compression means (SS) of the coupling means (S1) of the syringe (S) push the first membrane (31) to its open position, thus freeing the passage through the first membrane (31).

Successively, the second coupling means (M2) of the safety device (100) are connected to the coupling means (D1) (Fig. 6) of a user device (D) disposed downstream the safety device (100) and connected to the bottle, the vial or the bag. In view of the above, the cannula (25) is disposed in operating position, and the second membrane (32) is pushed by the cannula (25) to its open position, thus freeing its passage. In such a case, the two membranes (31, 32) are in open position (Fig. 4) and therefore, by pulling a plunger (ST) of the syringe (S) backwards, it is possible to take the liquid contained inside the bottle or the vial or the bag connected to said user device (D).

After taking the liquid, which is introduced in the syringe (S), the safety device (100) is disconnected from the user device (D) and, consequently, the cannula (25) is disposed in non-operating position, and the second membrane (32) is disposed in closed position, so that the liquid is prevented from leaking from the duct (10) of the safety device (100).

In such a way, the syringe (S) and the safety device (100), which are connected one to another, can be transported from the point of taking/preparation to a point of administration/infusion in a safe way.

After transporting the syringe (S) and the safety device (100) to the point of administration/infusion, the second coupling means (M2) of the safety device (100) are coupled with coupling means (D1) of another user device (D) connected to a receptacle (Q) (Fig. 7) with the pharmacological fluid to be administered/infused.

The coupling of the second coupling means (M2) of the safety device (100) with the coupling means (D1) of the user device (D) disposes the cannula (25) in operating position, consequently positioning the second membrane (32) in open position. In view of the above, the syringe and the user device (D) are hydraulically connected by means of the safety device and, when pushing the plunger (ST) of the syringe, the liquid contained in the syringe (S) will pass in the axial duct (10) of the safety device, then in the axial duct (D10) of the user device (D), being finally poured in the receptacle (Q) connected to the user device (D).

After emptying the syringe (S), the syringe (S) can be uncoupled from the safety device (100) (see Fig. 7) by uncoupling the coupling means (S1) of the syringe (S) and the first coupling means (M1) of the safety device (100).

In such a case, the first membrane (31) is disposed in closed position, closing the passage and preventing the liquid from leaking from the axial duct (10) of the safety device (100).

The advantages of the present invention are manifest after the preceding description.

In particular, the safety device (100) generates a double safety in both flowing directions of the liquid. In fact, the safety device (100) permits the passage of liquid inside the axial duct (10), during the taking and during the administration, only if a syringe (S) and the user device (D) are coupled with the safety device (100).

When the safety device (100) is not connected with an element disposed upstream or downstream, i.e. when the coupling means (M1, M2) of the safety device (100) are not connected with coupling means (S1, D1) of another element, then the liquid is prevented from leaking because one of the two membranes (31, 32), which act as safety valves, is closed.

Therefore, the liquid (chemotherapeutic medicine or toxic liquids) will not leak from the axial duct (10) in case of uncoupling of the coupling means (M1, M2) because of the fact that, also in this condition, a tight compartment is determined by the closure of one of said two membranes (31, 32).

In conclusion, the safety device (100) permits to transport syringes and injection systems in a very safe way, avoiding leaks of hazardous medicine from the syringe or from the injection system that may be dispersed in the environment or go in direct contact with a patient or with an operator with severe consequences for their health.

Although the coupling means (M1, M2, S1, D1) illustrated in the appended figures comprise dual-lock connectors. said coupling means may comprise magnetic coupling means, bayonet coupling means or the like.

## Claims

1. Safety assembly (200) for the reconstitution, the taking or the infusion of pharmacological liquids, comprising:
- a needle-less syringe (S), comprising an axial duct (S10) in communication with an opening, and coupling means (S1) in correspondence of said opening; and
- a safety device (100) comprising:
- first coupling means (M1) configured in such a way to be coupled with said coupling means (S1) of the syringe (S) in such a way to fix the safety device to the syringe, second coupling means (M2),
- an axial duct (10), that extends from the first coupling means (M1) to the second coupling means (M2), and
- a first and a second membrane (31, 32) disposed inside said axial duct (10); each membrane (31, 32) being provided with a through hole (F1, F2) and being in a non-compressed closed position wherein the hole of the membrane is closed and obstructs the passage of liquid in the axial duct, and in a compressed open position, wherein the hole of the membrane is open and does not obstruct the passage of liquid in the axial duct (10); each membrane (31, 32) having a channel (310, 320) in communication with said through hole (F1, F2);
said safety assembly (200) also comprises:
- first compression means (SS) associated with said coupling means (S1) of the syringe to compress the first membrane (31) to the compressed open position, when the coupling means (S1) of the syringe are coupled with said first coupling means (M1) of the safety device (100); and
- second compression means (2) comprising a cannula (25) slidingly mounted into the axial duct (10) of the safety device (100) to compress the second membrane (32) in the compressed open position; said cannula (25) comprising an ending portion (22) and a tip (21);
**characterized in that**
said safety assembly (200) also comprises a user device (D) comprising an axial duct (D10) in communication with an opening and coupling means (D1) in correspondence of said opening, said user device (D) having a needle (D2) for the injection of a pharmacological fluid to a user or being connected to a receptacle (Q) for the administration/infusion of the pharmacological fluid;
the safety device (100) comprising the second coupling means (M2) configured in such a way to be coupled with the coupling means (D1) of the user device (D) in such a way to fix the safety device to the user device;
when said coupling means (D1) of the user device are coupled with the second coupling means (M2) of the safety device, the ending portion (22) of the cannula (25) is coupled by interference inside the axial duct (D10) of the user device (D) and the tip (21) of the cannula is suitable for pushing said second membrane (32) to its compressed open position, enlarging the through hole (F2) of the second membrane (32), without crossing the channels (320, 321) of the membranes (31, 32).

2. The safety assembly (200) of claim 1, wherein said coupling means (S1) of the syringe (S) comprise a male luer-lock connector; said coupling means (D1) of the user device (D) comprise a female luer-lock connector; said first coupling means (M1) of the safety device (100) comprise a female luer-lock attachment; said second coupling means (M2) of the safety device (100) comprise a male luer-lock connector.

3. The safety assembly (200) of any one of the preceding claims, wherein said first compression means (SS) comprise a tip of the coupling means (S1) of the syringe (S) suitable for pushing said first membrane (31) to the open position, when said coupling means (S1) of the syringe (S) are coupled with said second coupling means (S2) of the safety device (100).

4. The safety assembly (200) of any one of the preceding claims, wherein said membranes (31, 32) are shaped like a semi-body and are disposed in opposite position in mutual contact.

5. The safety assembly (200) of any one of the preceding claims, wherein said safety device (100) is provided along said axial duct (10) with a seat (1a) for said two membranes (31, 32).

6. The safety assembly (200) of any one of the preceding claims, wherein said safety device (100) comprises a lid (4) with a first portion (41) inserted in the axial duct (10) of the safety device (100), and a second portion (42) comprising said first coupling means (M1) of the safety device (100).

7. The assembly of claim 6 when depending on claim 5, wherein said seat (1a) that houses the two membranes (31, 32) is defined by the first portion (41) of the lid (4) and by a portion of the axial duct (10) of the safety device (200).

8. The assembly of any one of the preceding claims, wherein said second membrane (32) pushes said cannula (25) to its non-operating position.

9. The assembly of any one of the preceding claims, wherein said safety device (100) comprises stop means (B) suitable for stopping said cannula (25) in its non-operating position; said stop means (B) comprising at least one stop tooth (B1) obtained in the longitudinal duct (10) and a radial tab (B2) obtained in said cannula (25); said radial tab (B2) being suitable for being stopped against said stop tooth (B1) when said cannula (2) is in said non-operating position.

## Patentansprüche

1. Sicherheitsanordnung (200) zur Rekonstitution, Entnahme oder Infusion von pharmakologischen Flüssigkeiten, umfassend:
- eine Spritze (S) ohne Nadel, umfassend eine axiale Leitung (S10), die mit einer Öffnung in Verbindung steht, und Kopplungsmittel (S1) an der Öffnung; und
- eine Sicherheitsvorrichtung (100), umfassend:
- erste Kopplungsmittel (M1), die so konfiguriert sind, dass sie mit den Kopplungsmitteln (S1) der Spritze (S) so gekoppelt werden, dass sie die Sicherheitsvorrichtung an der Spritze befestigen,
- zweite Kopplungsmittel (M2),
- eine axiale Leitung (10), die sich von den ersten Kopplungsmitteln (M1) zu den zweiten Kopplungsmitteln (M2) erstreckt, und
- eine erste und eine zweite Membran (31, 32), die in der axialen Leitung (10) angeordnet sind; wobei jede Membran (31, 32) ein Durchgangsloch (F1, F2) aufweist und sich in einer nicht komprimierten Schließstellung befindet, in der das Loch der Membran geschlossen ist und der Flüssigkeitsdurchfluss in der axialen Leitung unterbunden wird, und in einer komprimierten Offenstellung, in der das Loch in der Membran geöffnet ist und der Flüssigkeitsdurchfluss in der axialen Leitung (10) nicht unterbunden wird; wobei jede Membran (31, 32) einen Kanal (310, 320) aufweist, der mit dem Durchgangsloch (F1, F2) in Verbindung steht;
wobei die Sicherheitsanordnung (200) ferner umfasst:
- erste Kompressionsmittel (SS), die mit den Kopplungsmitteln (S1) der Spritze verbunden sind, um die erste Membran (31) in der komprimierten Offenstellung zu komprimieren, wenn die Kopplungsmittel (S1) der Spritze mit den ersten Kopplungsmitteln (M1) der Sicherheitsvorrichtung (100) gekoppelt sind; und
- zweite Kompressionsmittel (2), umfassend eine Kanüle (25), die gleitbeweglich in der axialen Leitung (10) der Sicherheitsvorrichtung (100) eingebaut ist, um die zweite Membran (32) in der komprimierten Offenstellung zu komprimieren; wobei die Kanüle (25) einen Endabschnitt (22) und eine Spitze (21) umfasst;
**dadurch gekennzeichnet, dass**
die Sicherheitsanordnung (200) ferner eine Benutzervorrichtung (D) umfasst, die eine axiale Leitung (D10) umfasst, die mit einer Öffnung und Kopplungsmitteln (D1) an der Öffnung in Verbindung steht, wobei die Benutzervorrichtung (D) eine Nadel (D2) zum Injizieren einer pharmakologischen Flüssigkeit in einen Benutzer umfasst oder mit einem Behälter (Q) zur Verabreichung/Infusion der pharmakologischen Flüssigkeit verbunden ist;
wobei die Sicherheitsvorrichtung (100) die zweiten Kopplungsmittel (M2) umfasst, die so konfiguriert sind, dass sie mit den Kopplungsmitteln (D1) der Benutzervorrichtung (D) so gekoppelt werden, dass sie die Sicherheitsvorrichtung an der Benutzervorrichtung befestigen;
wenn die Kopplungsmittel (D1) der Benutzervorrichtung mit den zweiten Kopplungsmitteln (M2) der Sicherheitsvorrichtung gekoppelt sind, ist der Endabschnitt (22) der Kanüle (25) durch Presspassung im Inneren der axialen Leitung (D10) der Benutzervorrichtung gekoppelt und die Spitze (21) der Kanüle ist in der Lage, die zweite Membran (32) in ihre komprimierte Offenstellung zu drücken, wobei sie das Durchgangsloch (F2) der zweiten Membran (32) erweitert, ohne die Leitungen (320, 321) der Membranen (31, 32) zu durchqueren.

2. Sicherheitsanordnung (200) nach Anspruch 1, wobei die Kopplungsmittel (S1) der Spritze (S) ein Luer-Verschluss-Außenverbindungsstück umfassen; wobei die Kopplungsmittel (D1) der Benutzervorrichtung (D) ein Luer-Verschluss-Innenverbindungsstück umfassen; wobei die ersten Kopplungsmittel (M1) der Sicherheitsvorrichtung (100) ein Luer-Verschluss-Innenverbindungsstück umfassen; wobei die zweiten Kopplungsmittel (M2) der Sicherheitsvorrichtung (100) ein Luer-Verschluss-Außenverbindungsstück umfassen.

3. Sicherheitsanordnung (200) nach einem der vorstehenden Ansprüche, wobei die ersten Kompressionsmittel (SS) eine Spitze der Kopplungsmittel (S1) der Spritze (S) umfassen, die in der Lage ist, die erste Membran (31) in ihre Offenstellung zu drücken, wenn die Kopplungsmittel (A1) der Spritze (S) mit den zweiten Kopplungsmitteln (S2) der Sicherheitsvorrichtung (100) gekoppelt sind.

4. Sicherheitsanordnung (200) nach einem der vorstehenden Ansprüche, wobei die Membranen (31, 32) halbschalenförmig sind und einander gegenüberliegend in Anschlag gehend angeordnet sind.

5. Sicherheitsanordnung (200) nach einem der vorstehenden Ansprüche, wobei die Sicherheitsvorrichtung (100) entlang der axialen Leitung (10) mit einem Sitz (1a) für die beiden Membranen (31, 32) versehen ist.

6. Sicherheitsanordnung (200) nach einem der vorstehenden Ansprüche, wobei die Sicherheitsvorrichtung (100) einen Deckel (4) umfasst mit einem ersten Abschnitt (41), der in die axiale Leitung (10) der Sicherheitsvorrichtung (100) eingesteckt ist, und mit einem zweiten Abschnitt (42), der die ersten Kopplungsmittel (M1) der Sicherheitsvorrichtung (100) umfasst.

7. Anordnung nach Anspruch 6, sofern abhängig von Anspruch 5, wobei der Sitz (1a), der die beiden Membranen (31, 32) aufnimmt, durch den ersten Abschnitt (41) des Deckels (4) und durch einen Abschnitt der axialen Leitung (10) der Sicherheitsvorrichtung (100) begrenzt wird.

8. Anordnung nach einem der vorstehenden Ansprüche, wobei die zweite Membran (32) die Kanüle (25) in ihre Nichtarbeitsstellung drückt.

9. Anordnung nach einem der vorstehenden Ansprüche, wobei die Sicherheitsvorrichtung (100) Anschlagmittel (B) umfasst, die in der Lage sind, die Kanüle (25) in ihrer Nichtarbeitsstellung zu stoppen; wobei die Anschlagmittel (B) mindestens einen Anschlagzahn (B1) umfassen, der in der Längsleitung (10) herausgearbeitet ist, und eine radiale Rippe (B2), die auf der Kanüle (25) herausgearbeitet ist; wobei die radiale Rippe (B2) dazu bestimmt ist, gegen den Anschlagzahn (B1) in Anschlag zu gehen, wenn die Kanüle (25) sich in ihrer Nichtarbeitsstellung befindet.

## Revendications

1. Groupe de sécurité (200) pour la reconstitution, le prélèvement ou l'infusion de fluides pharmacologiques, comprenant :
- une seringue (S), sans aiguille, comprenant un conduit axial (S10) qui communique avec une embouchure et des moyens d'accrochage (S1) en correspondance de ladite embouchure ; et
- un dispositif de sécurité (100) comprenant :
- des premiers moyens d'accrochage (M1) configurés de manière à s'accoupler avec lesdits moyens d'accrochage (S1) de la seringue (S) afin de bloquer le dispositif de sécurité à la seringue,
- des seconds moyens d'accrochage (M2),
- un conduit axial (10) qui se déploie depuis les premiers moyens d'accrochage (M1) aux seconds moyens d'accrochage (M2), et
- une première et une seconde membrane (31, 32) disposées dans ledit conduit axial (10) ; chaque membrane (31, 32) ayant un orifice passant (F1, F2) et assumant une position non comprimée de fermeture, où l'orifice de la membrane est fermé en obstruant le passage de liquide dans le conduit axial, et une position comprimée d'ouverture, où l'orifice de la membrane est ouvert et n'obstrue pas le passage de liquide dans le conduit axial (10) ; chaque membrane (31, 32) ayant un canal (310, 320) qui communique avec ledit orifice passant (F1, F2) ;
ledit groupe de sécurité (200) comprend également :
- des premiers moyens de compression (SS) associés aux susdits moyens d'accrochage (S1) de la seringue qui compriment la première membrane (31) sur la position comprimée d'ouverture lorsque les moyens d'accrochage (S1) de la seringue sont couplés aux susdits premiers moyens d'accrochage (M1) du dispositif de sécurité (100) ; et
- des seconds moyens de compression (2) comprenant une canule (25) montée coulissante à l'intérieur du conduit axial (10) du dispositif de sécurité (100) pour comprimer la seconde membrane (32) sur la position comprimée d'ouverture ; ladite canule (25) comprenant une portion d'extrémité (22) et une pointe (21) ;
**caractérisé en ce que**
ledit groupe de sécurité (200) comprend également un dispositif utilisateur (D) comprenant un conduit axial (D10) qui communique avec une embouchure et des moyens d'accrochage (D1) en correspondance de ladite embouchure ; ledit dispositif utilisateur (D) comprend une aiguille (D2) pour l'injection d'un fluide pharmacologique à un patient ou, étant relié à un récipient (Q), pour la distribution/infusion du fluide pharmacologique ;
le dispositif de sécurité (100) comprend les seconds moyens d'accrochage (M2) configurés de manière à se coupler avec les moyens d'accrochage (D1) du dispositif utilisateur (D), afin de bloquer le dispositif de sécurité au dispositif utilisateur ;
lorsque les susdits moyens d'accrochage (D1) du dispositif utilisateur sont couplés aux seconds moyens d'accrochage (M2) du dispositif de sécurité, la portion d'extrémité (22) de la canule (25) est couplée par interférence dans le conduit axial (D10) du dispositif utilisateur et la pointe (21) de la canule est apte à pousser ladite seconde membrane (32) sur sa position comprimée d'ouverture, en dilatant l'orifice central (F2) de la seconde membrane (32), sans traverser les conduits (320, 321) des membranes (31, 32).

2. Groupe de sécurité (200) selon la revendication 1, où lesdits moyens d'accrochage (S1) de la seringue (S) comprennent un embout Luer-Lock mâle ; lesdits moyens d'accrochage (D1) du dispositif utilisateur (D) comprennent un embout Luer-Lock femelle ; lesdits premiers moyens d'accrochage (M1) du dispositif de sécurité (100) comprenant un embout Luer-Lock femelle ; lesdits seconds moyens d'accrochage (M2) du dispositif de sécurité (100) comprenant un embout Luer-Lock mâle.

3. Groupe de sécurité (200) selon l'une quelconque des revendications précédentes, où lesdits premiers moyens de compression (SS) comprennent une pointe des moyens d'accrochage (S1) de la seringue (S) apte à pousser ladite première membrane (31) sur sa position d'ouverture lorsque lesdits moyens d'accrochage (S1) de la seringue (S) sont couplés avec lesdits seconds moyens d'accrochage (S2) du dispositif de sécurité (100).

4. Groupe de sécurité (200) selon l'une quelconque des revendications précédentes, où lesdites membranes (31, 32) ont la forme d'une semi-coque et sont disposées en butée et opposées entre elles.

5. Groupe de sécurité (200) selon l'une quelconque des revendications précédentes, où ledit dispositif de sécurité (100) comprend, le long du susdit conduit axial (10), un emplacement (1a) pour lesdites deux membranes (31, 32).

6. Groupe de sécurité (200) selon l'une quelconque des revendications précédentes, où ledit dispositif de sécurité (100) comprend un couvercle (4) avec une première portion (41), enfilée dans le conduit axial (10) du dispositif de sécurité (100), et une seconde portion (42) comprenant lesdits premiers moyens d'accrochage (M1) du dispositif de sécurité (100).

7. Groupe selon la revendication 6 quand dépendante de la revendication 5, où ledit emplacement (1a) dans lequel les deux membranes (31, 32) sont logées est délimité par la première portion (41) du couvercle (4) et par une portion du conduit axial (10) du dispositif de sécurité (100).

8. Groupe selon l'une quelconque des revendications précédentes, où ladite seconde membrane (32) pousse ladite canule (25) sur sa position non opérationnelle.

9. Groupe selon l'une quelconque des revendications précédentes, où ledit dispositif de sécurité (100) comprend des moyens d'arrêt (B) aptes à arrêter ladite canule (25) sur sa position non opérationnelle ; lesdits moyens d'arrêt (B) comprenant au moins une dent de butée (B1) réalisée dans le conduit longitudinal (10) et une ailette radiale (B2) réalisée sur ladite canule (25) ; ladite ailette radiale (B2) étant destinée à aller en butée contre ladite dent de butée (B1) quand ladite canule (25) se trouve sur ladite position non opérationnelle.
